# EUROPEAN PATENT SPECIFICATION

(11) **EP 2 738 399 B1**
(45) Date of publication and mention of the grant of the patent: **10.05.2017**
(21) Application number: 13191352.7
(22) Date of filing: 04.11.2013
(51) Int. Cl.: F16B 7/20, F16B 21/04

(54) **Connector**
Steckverbinder
Connecteur

(30) Priority: 30.11.2012 GB 201221576
(43) Date of publication of application: 04.06.2014
(73) Proprietor: Aber Instruments Ltd, Ceredigion SY23 3AH (GB)
(72) Inventor: Agate, Lindsay, Llanon, Ceredigion SY23 5HH (GB)
(74) Representative: Baker, Thomas Edward

(56) References cited:
- EP-A1- 0 288 181
- WO-A1-00/45469
- US-A1- 2007 281 532

## Description

The present invention relates to a connector that beneficially enables connection between a device that is typically poorly supported, meaning that when the connector is brought into contact with the device there can be little force applied to the device in order that it does not move. An example of such a device is a flexible disposable bioreactor wherein a probe is secured within a wall of the bioreactor and a connection is required to enable a reading to be taken from the electrodes of the probe.
Single use or disposable biomass monitoring apparatus are presently utilised which comprise an enclosure provided for containing a biological medium and a probe or sensing device for making an impedance measurement of the medium. Accordingly, a sensing device or probe is fixed through a wall of the enclosure in such a way to form a seal and ensure there is no leakage. The enclosure itself can be a flexible polymeric material meaning that the enclosure itself does not form a rigid structure. This can be effectively visualised as a flexible plastic bag. This results in a problem when the enclosure is positioned for use a connector must be connected to the sensing device in order for the capacitance measurement to be transferred from the electrode of the sensing device to the monitoring device. Accordingly, the connector must be fixed to the sensing device. However, when a force is applied to the sensing device and thus the enclosure the flexible nature of the enclosure means that it is difficult to secure a connector to the necessary measurement device.

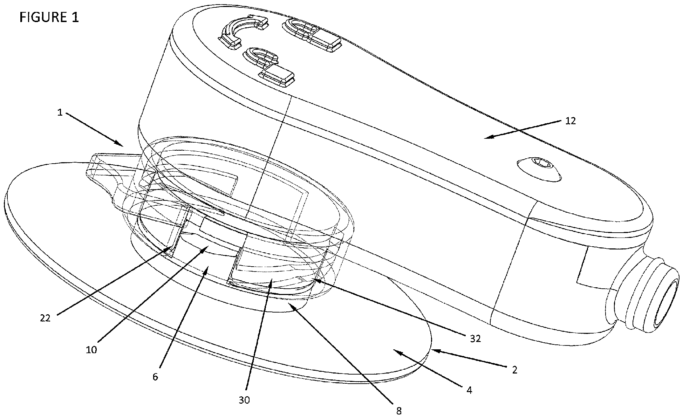

Figure 1 shows a schematic representation of an arrangement devised by the applicant to overcome the above mentioned problem. Referring to Figure 1, the connector (1) connects to a sensing device (2). On the underside of this sensing device (2) four electric probes are provided which extend into the enclosure for receiving electrical information in order that capacitance can be calculated from a biological medium. This sensing device is secured in the wall of a flexible enclosure typically via an adhesive and thus forms a part of the wall of the enclosure and ensures that there is no leakage of the biological material from the enclosure. In the arrangement shown the sensing device comprises a plate like disk (4) to be secured to and in the surface of the enclosure. Four probes are provided on a moulded projection extending outwardly from the plane of the underside of the sensing device. Shown in Figure 1 is the rearward portion of the sensing device. Visible in Figure 1 is a sensing device ear (6) protruding in the longitudinal axis of the sensing device secured to a support (8). Diametrically opposing sensing device ears (6) are provided substantially on opposite sides of the support (8). The ears include a lateral protrusion (10). Between the ears (6) sit (not visible in Figure 1) four connections extending from the electrodes which extend into the enclosure. These electrodes extend through the connector and a signal is taken from the electrodes to a monitoring device where capacitance of the medium under measurement is determined.

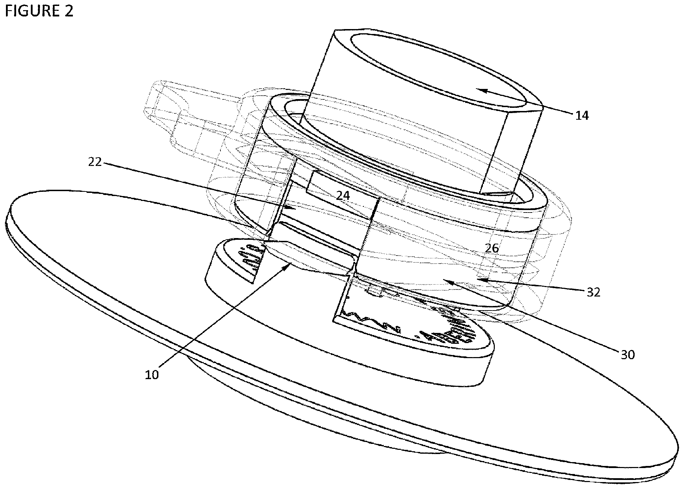

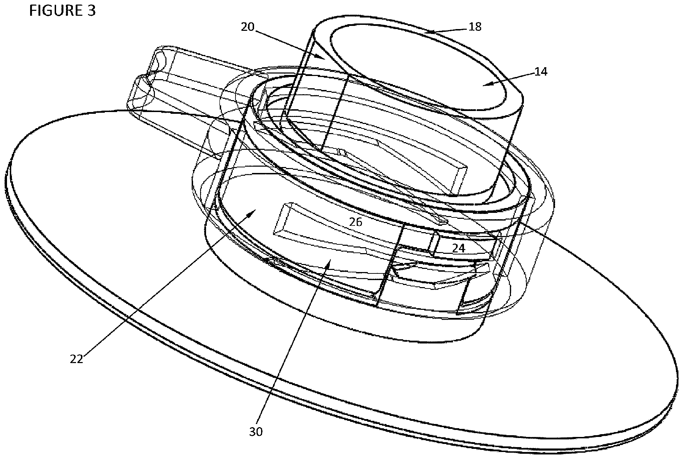

Referring now to the connector (1) with reference also to Figures 2 and 3, the connector comprises a body (12) which provides an aperture (not shown) into which an interface arrangement (14) seats. In Figure 1, the interface arrangement is hidden behind the collar (16) and a further part is hidden within the body (12). Referring to Figures 2 and 3, the interface arrangement (14) comprises a rearward section (18) which seats into an opening provided in the body (12). Opposing sides of the rearward section (18) indicated by reference numeral (20) are chamfered and are received by a corresponding shaped opening in the body (12). This rearward section (18) is therefore received into the body (12) and is fixedly secured therein via friction joint.

During assembly, the interface arrangement (14) seats into the collar (16) and the rearward section (18) is then secured to the body (12). In this manner the collar (16) is effectively retained between the interface arrangement (14) and the body (12). The collar may, however, rotate relative to the body (12) and the interface arrangement (14). The collar (16) comprises a recess (22) best seen in Figures 2 and 3 which is provided cut away from the radially inward surface of the collar. As clearly visible in Figures 2 and 3, this recess (22) in the collar (16) provides an opening for receipt of the diametrically opposing lateral protrusions (10) of the sensing device, and the interface arrangement (14) including a cutaway portion (42) defines a space for receipt of the ear (6). During assembly the recess (22) also receives an ear (24) projecting radially outwardly from the interface arrangement (14). As the interface arrangement (14) is received into the collar (16) the ear (24) seats into the recess (22) of the collar (16) and butts up against the ledge or shoulder defining the base of the recess. In communication with the recess (22) is a first channel (26) meaning that as the collar (16) is rotated relative to the interface arrangement (14) and body (12), the ear (24) of the interface arrangement is effectively guided by the channel (26). This can be shown by comparing Figure 2 and Figure 3 where in Figure 2 the ear (24) is shown provided in the recess (22). However, when the collar (16) is rotated as shown in Figure 3, the collar (16) is moved relative to the ear (24) along the first channel (26). It is important to note that it is the collar (16) that is rotating whereas the interface arrangement (14) remains stationary. The provision of the ear (24) moving through the first channel (26) ensures the accurate maintaining of the axial position of the collar (16) relative to the interface arrangement (14).
It is further noted that the collar (16) comprises a user operable handle (28) enabling ease of use for a user to rotate the collar between a first configuration enabling releasable engagement with a sensing device and a second configuration wherein a sensing device is restrained by the collar shown in Figure 3.
Further provided in communication with the recess (22) provided in the collar (16) is a second channel (30). The second channel (30) is provided in the radially inwardly surface of the collar (16) and generally extends circumferentially around the inner surface of the collar (16). A portion of the second channel (30) identified by reference numeral (32) extends generally parallel to the first channel (26). A second portion of the second channel (30) extends from the recess (22) not perpendicular to the longitudinal axis of the collar. This configuration is provided such that as the lateral protrusion (10) of the sensing device projecting radially outwardly is located into the recess (22) of the collar (16) and the collar (16) is rotated, the lateral protrusion (10) is drawn along the second portion of the second channel (30) and the angle provided in the second portion of the second channel (30) means that the sensing device is drawn into closer communication with the interface arrangement (14).
Figure 2 shows a first configuration which enables releasable engagement of the sensing device with the interface arrangement (14). The collar (16) and in particular the handle (28) is rotated in a clockwise direction which then causes second channel (30) to be drawn along the lateral protrusion (10) thus moving to the second configuration whereby the sensing device is restrained by the collar (16). In such a configuration the sensing device cannot be withdrawn from the interface arrangement (14) due to the lateral protrusion (10) and second lateral protrusion (10) provided diametrically opposite which are restrained and effectively retained in the second channel (30). The sensing device is then secured to the connector (1) meaning that information from the enclosure from the electrodes through to connector (1) can be achieved.

D1 (WO00/45469) discloses a sealed connector assembly (10) for use with equipment subject to submersion under water and includes two electrical connector shells (25, 29) which are inserted in bayonet fashion into opposite sides of a locking member (40), which is then manually rotated to lock the parts together and shield the electrical connection. The three parts have alignment marks (56, 58, 59) for axial assembly and another mark (57) indicating the rotation of the locking member (40) to a locked position. A body of grease (55) is disposed in one connector shell (25) and an O-ring (50) is placed on the barrel (27) to provide sealing when the connector shells (25, 27) are locked together. The connector shells (25, 29) have wire entry ports and sealing ports for admitting an encapsulating material to seal the wire entry ends. Score lines (68, 69) allow for fracture and removal of the locking member (40) during servicing. Methods of assembly and disassembly are also disclosed.

In operation, however, there are deficiencies associated with known arrangements. In use the aim is to ensure that the connector (1) can be positioned in communication with the sensing device whilst transferring minimal pressure onto the sensing device and it is desirable that connection can be achieved with minimal effort. The present invention overcomes the deficiencies associated with this known arrangement.

According to the present invention there is a connector as defined in claim 1.

An effective solution has been provided to the significant problem of the difficulty when attempting to rotate the collar (16) to secure the ears of a sensing device into the second channel (30). This has been achieved through the provision of the shoulder configured to limit insertion of at least a portion of in particular the ear of the sensing device. This shoulder substantially causes alignment of at least a portion of an ear of a sensing device with the second channel ensuring positive initial engagement of the connector (1) with the sensing device and then enables ease of rotation of the collar (16) to secure the sensing device relative to the connector (1).

The shoulder beneficially causes a reduction in the circumferential length of the recess. The recess according to the present invention has a circumferential length which is effectively reduced by the provision of the shoulder moving into the recess. The recess therefore beneficially has a further first circumferential length defining an opening and a second reduced circumferential length caused by the provision of the shoulder.

The circumferential length of a first and/or second channel is less than 50% of the inner circumference of the collar, and is preferably less than 30% of the inner circumference of the collar.

The shoulder beneficially extends circumferentially and provides a substantial planar device contact surface.

The ear of the interface arrangement beneficially seats into the recess of the collar. Furthermore, the ear of the interface beneficially seats into the narrowed portion of the recess beyond the shoulder.

The recess is arranged to limit axial insertion of the interface arrangement into the collar. The recess beneficially limits axial insertion of the ear of the interface arrangement into the collar. The interface arrangement beneficially comprises a second ear extending radially outwardly. The second ear is beneficially substantially diametrically opposite the first ear.

Also according to the present invention there is a method of providing a connection to a device as defined in claim 9.

The present invention will now be described by way of example only with reference to the accompanying drawings, where:
Figures 1, 2 and 3 show a prior art connector connecting to a sensing device. Figure 1 includes the connector body (12), Figure 2 shows initial contact between the connector and sensing device, and Figure 3 shows the sensing device in the secured configuration.

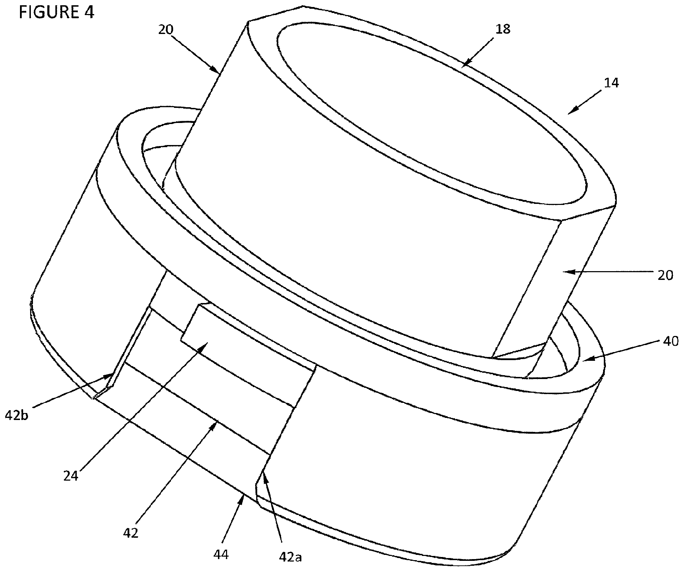

Figure 4 is a schematic perspective view of an interface arrangement according to an exemplary embodiment of the present invention.

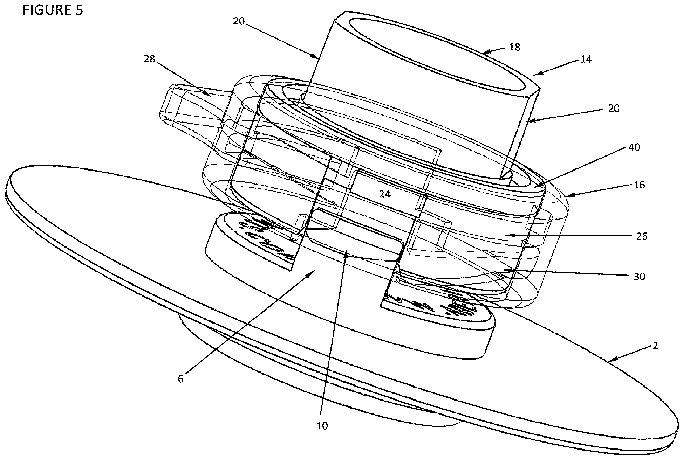

Figure 5 is a schematic perspective view of the interface arrangement and collar wherein the connector is in communication with a sensing device in a first configuration enabling releasable engagement.

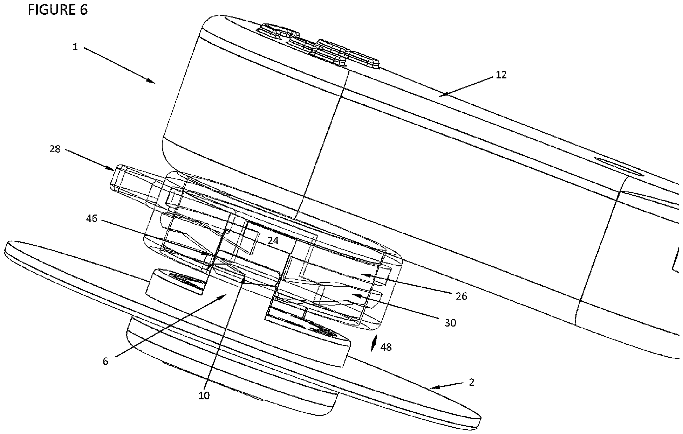

Figure 6 shows the same configuration as Figure 5 but with the body (12).

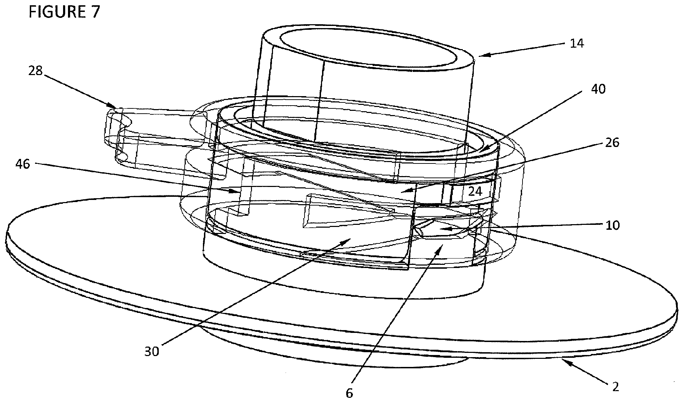

Figure 7 shows the collar in the second configuration wherein the sensor device is restrained by the collar.

Referring to Figure 4, the interface arrangement (14) is shown according to a part of an embodiment of the present invention. The rearward section (18) is the same as the prior art arrangement. It is clear in this figure that a shoulder (40) is provided which when the connector is assembled abuts against the body (12). This means that the collar (16) is sandwiched between the interface arrangement (14) and in particular the ears (24) when in the first configuration and the body (12). Also as in the prior art arrangement, there is a cutaway portion (42) cut into the peripheral edge extending from a first end (44) of the interface arrangement (14) to the ear (24). This cutaway portion, which beneficially has the corresponding same cutaway portion diametrically opposite, is for receipt of the distal end of the ear (6) of the sensing device. This cutaway (42) is substantial planar in the longitudinal axis and the radially inner surface of the ear of the sensing device abuts this surface. The difference between the interface arrangement (14) as shown in Figure 4 and the prior art arrangement is the reduction in the circumferential length of the ear (24). In the prior art arrangement this ear (24) extends circumferentially to the same circumferential length as the cutaway (42). However, in the present invention this length has been reduced. As will be shown with respect to the remaining Figures, this enables the ear (24) to seat into the recess (22) of the collar (16) without interference from the shoulder (46).
Now referring to Figures 6 and 7, where Figure 6 shows the connector in a first configuration in initial communication with a sensing device, and Figure 7 shows the connector in a second configuration where the device is restrained by the collar, like components with respect to the prior art arrangements have the same reference numerals. The cutaway (42) defined between circumferential cutaway ends (42a, 42b) is shaped to receive the corresponding ears (6) of the sensing device. This cutaway (42) therefore prevents in use the sensing device from rotating as the interface arrangement (14) is secured to the body which is designed not to be moved when the collar is rotated between the first configuration which allows releasable engagement with the sensing device and the second configuration wherein the device is restrained by the collar. Accordingly, the collar (16) is the component that rotates. Both Figures 6 and 7, clearly identify the shoulder (46) which enables a significant increase in functionality for an operator.
Referring to Figures 5, 6 and 7, Figures 5 and 6 show the same configuration without and with the body (12). The connector (1) is brought towards the sensing device (2) and the ears (6) of the sensing device (2) are located into the recess (22) of the collar and the corresponding cutaway (42) of the interface arrangement (14). The problem with the prior art arrangement is the insertion of the ear (6) of the sensing device (2) has to be the correct amount in order to align the lateral protrusion (10) of the ear (6) with the second channel (30). The problem is clearly identified in Figure 1 wherein under normal circumstances and operating conditions it is usual that the ears (6) are inserted too far meaning that the lateral protrusion (10) does not align with the second channel (30). According to the present invention, however, the leading edge or distal end of the ear (6) of the sensing device (2) comes into communication with the shoulder (46). Further insertion is prevented. This is clearly shown in Figure 6 where it is clear that there is a separation between the interface arrangement (14) and the sensing device (2). This separation is identified by arrow (48). It will be noted, however, that the lateral protrusion (10) of the sensing device (2) is aligned with the second channel (30) meaning that clockwise rotation of the collar (16) draws the second channel (30) around the lateral protrusion (10). As it does so, it effectively draws the rearward portion of the sensing device having the electrodes projecting therefrom into communication with corresponding apertures in the interface arrangement (14) and thus ensures a solid connection between the sensing device (2) and the interface arrangement (14).

It will be appreciated that the reduced circumferential length of the ear (24) of the interface arrangement (14) seats into the reduced circumferential length portion of the recess (22). This ear (24) aligns with the first channel (26) and thus as shown in Figure 7 where the collar (16) has been rotated the ear (24) is securely positioned in the first channel (26) and the lateral protrusion (10) is securely positioned in the second channel (30). It will be appreciated that the lateral protrusion (10) of the sensing device (2) is shaped along with shape of the second channel (30) to ensure smooth guiding along the second channel (30) to enable secure communication between the interface arrangement (14) and the sensing device (2).

The present invention provides an effective solution to the problems associated with the prior art arrangement that significantly improves usability as it takes away any user requirement for precision in respect of the degree of insertion of the ear (6) into the recess (22) provided in the collar (16) and the corresponding cutaway portion (42) in the interface arrangement (14).

The present invention has been described by way of example only and it will be appreciated to the skilled addressee that modifications and variations may be made without departing from the scope of protection afforded by the appended claims.

## Claims

1. A connector comprising:
a device interface arrangement (14);
a collar (16) coaxially and rotatably mounted to the device interface arrangement (14);
wherein the collar (16) is arranged to rotate between a first configuration enabling releasable engagement with a device (4), and a second configuration wherein the device (4) is restrained by the collar (16), the collar (16) having a radial width defined between a radially outer surface and a radially inner surface;
the device interface arrangement (14) including an ear (24) extending radially outwardly, the ear arranged to locate into a recess (22) provided in the radially inner surface of the collar (16), the collar (16) further including a first channel extending circumferentially in the radially inner surface, the first channel being in communication with the recess (22) and being arranged such that as the collar (16) rotates relative to the device interface arrangement (14) between the first and second configuration the first channel receives the ear (24); **characterised in that** the recess (22) includes a shoulder (46) configured to limit insertion of at least a portion of the device (4) therein, wherein the shoulder (46) is arranged to substantially align the device (4) with a second channel provided in the radially inwardly surface of the collar (16), and rotation of the collar (16) between the first and second configurations enables the second channel to receive the device.

2. A connector according to claim 1, wherein the shoulder (46) causes a reduction in the circumferential length of the recess (22).

3. A connector according to any preceding claim, wherein the circumferential length of the first and/or second channels is less than 50% of the inner circumference of the collar, and is preferably less than 30% of the inner circumference of the collar.

4. A connector according to any preceding claim, wherein the shoulder (46) extends circumferentially and provides a substantially planar device contact surface.

5. A connector according to any preceding claim, wherein the ear (24) of the device interface arrangement (14) seats into the recess (22) of the collar (16).

6. A connector according to claim 5, wherein the recess (22) is arranged to limit axial insertion of the device interface arrangement (14) into the collar (16).

7. A connector according to any preceding claim, wherein the device interface arrangement comprises a second ear extending radially outwardly.

8. A connector according to claim 7, wherein the second ear is substantially diametrically opposite the first ear.

9. A method of providing a connection to a device (4) comprising providing a device interface arrangement (14) and a collar (16) coaxially and rotatably mounted to the device interface arrangement;
rotating the collar (30) between a first configuration enabling releasable engagement with a device (4), and a second configuration wherein the device (4) is restrained by the collar (30), the collar (30) having a radial width defined between a radially outer surface and a radially inner surface;
wherein the device interface arrangement includes an ear extending radially outwardly, the ear arranged to locate into a recess (22) provided in the radially inner surface of the collar (30), the collar (30) further including a first channel (26) extending circumferentially in the radially inner surface, the first channel (26) being in communication with the recess (22) and being arranged such that as the collar (30) rotates relative to the device interface arrangement between the first and second configuration the first channel (26) receives the ear; **characterised in that** the recess includes a shoulder (46) configured to limit insertion of a device therein, wherein the shoulder is arranged to substantially align a device with a second channel (30) provided in the radially inwardly surface of the collar (16), and rotation of the collar (16) between the first and second configurations enables the second channel (30) to receive the device (4).

## Patentansprüche

1. Verbindungselement, das Folgendes umfasst:
eine Vorrichtungsschnittstellenanordnung (14);
einen Kranz (16), der an der Vorrichtungsschnittstellenanordnung (14) koaxial und drehbar befestigt ist;
wobei der Kranz (16) dazu ausgelegt ist, zwischen einer ersten Konfiguration, die einen lösbaren Eingriff mit einer Vorrichtung (4) ermöglicht, und einer zweiten Konfiguration zu drehen, wobei die Vorrichtung (4) durch den Kranz (16) beschränkt ist, wobei der Kranz (16) eine radiale Breite, die zwischen einer radialen Außenfläche und einer radialen Innenfläche definiert ist, besitzt;
wobei die Vorrichtungsschnittstellenanordnung (14) eine Lasche (24) enthält, die sich radial auswärts erstreckt, wobei die Lasche dazu ausgelegt ist, sich in einer Aussparung (22) zu befinden, die in der radialen Innenfläche des Kranzes (16) vorgesehen ist, wobei der Kranz (16) ferner einen ersten Kanal enthält, der sich in der radialen Innenfläche in Umfangsrichtung erstreckt, wobei der erste Kanal mit der Aussparung (22) in Kommunikation ist und so angeordnet ist, dass der erste Kanal die Lasche (24) aufnimmt, während der Kranz (16) sich bezüglich der Vorrichtungsschnittstellenanordnung (14) zwischen der ersten und der zweiten Konfiguration dreht; **dadurch gekennzeichnet, dass** die Aussparung (22) eine Schulter (46) aufweist, die konfiguriert ist, das Einfügen mindestens eines Abschnitts der Vorrichtung (4) in sie zu begrenzen, wobei die Schulter (46) dazu ausgelegt ist, die Vorrichtung (4) auf einen zweiten Kanal auszurichten, der in der radialen Innenfläche des Kranzes (16) vorgesehen ist, und wobei durch eine Drehung des Kranzes (16) zwischen der ersten und der zweiten Konfiguration der zweite Kanal die Vorrichtung aufnehmen kann.

2. Verbindungselement nach Anspruch 1, wobei die Schulter (46) eine Verkürzung der Umfangslänge der Aussparung (22) bewirkt.

3. Verbindungselement nach einem der vorhergehenden Ansprüche, wobei die Umfangslänge des ersten Kanals und/oder des zweiten Kanals weniger als 50 % des inneren Umfangs des Kranzes beträgt und vorzugsweise weniger als 30 % des inneren Umfangs des Kranzes beträgt.

4. Verbindungselement nach einem der vorhergehenden Ansprüche, wobei sich die Schulter (46) in Umfangsrichtung erstreckt und eine im Wesentlichen ebene Vorrichtungskontaktfläche bereitstellt.

5. Verbindungselement nach einem der vorhergehenden Ansprüche, wobei sich die Lasche (24) der Vorrichtungsschnittstellenanordnung (14) in die Aussparung (22) des Kranzes (16) setzt.

6. Verbindungselement nach Anspruch 5, wobei die Aussparung (22) dazu ausgelegt ist, das axiale Einfügen der Vorrichtungsschnittstellenanordnung (14) in den Kranz (16) zu begrenzen.

7. Verbindungselement nach einem der vorhergehenden Ansprüche, wobei die Vorrichtungsschnittstellenanordnung eine zweite Lasche umfasst, die sich radial auswärts erstreckt.

8. Verbindungselement nach Anspruch 7, wobei die zweite Lasche der ersten Lasche im Wesentlichen diametral gegenüberliegt.

9. Verfahren zum Bereitstellen einer Verbindung mit einer Vorrichtung (4), das das Bereitstellen einer Vorrichtungsschnittstellenanordnung (14) und eines Kranzes (16), der an der Vorrichtungsschnittstellenanordnung koaxial und drehbar befestigt ist; und
das Drehen des Kranzes (30) zwischen einer ersten Konfiguration, die einen lösbaren Eingriff mit einer Vorrichtung (4) ermöglicht, und einer zweiten Konfiguration, umfasst, wobei die Vorrichtung (4) durch den Kranz (30) beschränkt ist, wobei der Kranz (30) eine radiale Breite, die zwischen einer radialen Außenfläche und einer radialen Innenfläche definiert ist, besitzt;
wobei die Vorrichtungsschnittstellenanordnung eine Lasche enthält, die sich radial auswärts erstreckt, wobei die Lasche dazu ausgelegt ist, sich in einer Aussparung (22) zu befinden, die in der radialen Innenfläche des Kranzes (30) vorgesehen ist, wobei der Kranz (30) ferner einen ersten Kanal (26) enthält, der sich in der radialen Innenfläche in Umfangsrichtung erstreckt, wobei der erste Kanal (26) mit der Aussparung (22) in Kommunikation ist und so angeordnet ist, dass der erste Kanal (26) die Lasche aufnimmt, während der Kranz (30) sich bezüglich der Vorrichtungsschnittstellenanordnung zwischen der ersten und der zweiten Konfiguration dreht; **dadurch gekennzeichnet, dass** die Aussparung eine Schulter (46) enthält, die konfiguriert ist, das Einfügen einer Vorrichtung in sie zu begrenzen, wobei die Schulter dazu ausgelegt ist, eine Vorrichtung auf einen zweiten Kanal (30) auszurichten, der in der radialen Innenfläche des Kranzes (16) vorgesehen ist, und wobei durch eine Drehung des Kranzes (16) zwischen der ersten und der zweiten Konfiguration der zweite Kanal (30) die Vorrichtung (4) aufnehmen kann.

## Revendications

1. Connecteur comprenant :
un agencement (14) d'interface de dispositif ;
un collier (16) monté coaxial et rotatif sur l'agencement (14) d'interface de dispositif, dans lequel le collier (16) est agencé pour tourner entre une première configuration permettant une entrée en prise amovible avec un dispositif (4), et une seconde configuration dans laquelle le dispositif (4) est retenu par le collier (16), le collier (16) ayant une largeur radiale délimitée entre une surface radialement extérieure et une surface radialement intérieure,
l'agencement (14) d'interface de dispositif comprenant une oreille (24) qui s'étend radialement vers l'extérieur, l'oreille étant agencée pour se situer dans un évidement (22) disposé dans la surface radialement intérieure du collier (16), le collier (16) comprenant en outre un premier canal qui s'étend suivant la circonférence de la surface radialement intérieure, le premier canal étant en communication avec l'évidement (22) et étant agencé de telle sorte que le collier (16) tourne par rapport à l'agencement (14) d'interface de dispositif entre les première et seconde configurations le premier canal reçoit l'oreille (24), **caractérisé en ce que** l'évidement (22) comprend un épaulement (46) configuré pour limiter l'introduction d'au moins une partie du dispositif (4) dans ledit évidement,
dans lequel l'épaulement (46) est agencé pour aligner sensiblement le dispositif (4) avec un second canal disposé dans la surface radialement intérieure du collier (16) et la rotation du collier (16) entre les première et seconde configurations permet au second canal de recevoir le dispositif.

2. Connecteur selon la revendication 1, dans lequel l'épaulement (46) entraîne une réduction de la longueur de la circonférence de l'évidement (22).

3. Connecteur selon l'une quelconque des revendications précédentes, dans lequel la longueur de la circonférence du premier et/ou du second canal est inférieure à 50 % de la circonférence intérieure du collier et est de préférence inférieure à 30 % de la circonférence intérieure du collier.

4. Connecteur selon l'une quelconque des revendications précédentes, dans lequel l'épaulement (46) s'étend suivant la circonférence et assure une surface de contact avec le dispositif sensiblement plane.

5. Connecteur selon l'une quelconque des revendications précédentes, dans lequel l'oreille (24) de l'agencement (14) d'interface de dispositif se met en place dans l'évidement (22) du collier (16).

6. Connecteur selon la revendication 5, dans lequel l'évidement (22) est agencé pour limiter l'introduction axiale de l'agencement (14) d'interface de dispositif dans le collier (16).

7. Connecteur selon l'une quelconque des revendications précédentes, dans lequel l'agencement d'interface de dispositif comprend une seconde oreille qui s'étend radialement vers l'extérieur.

8. Connecteur selon la revendication 7, dans lequel la seconde oreille est sensiblement diamétralement opposée à la première oreille.

9. Procédé pour assurer un raccordement avec un dispositif (4), comprenant :
fournir un agencement (14) d'interface de dispositif et un collier (16) monté coaxial et rotatif sur l'agencement d'interface de dispositif ;
faire tourner le collier (30) entre une première configuration permettant une entrée en prise amovible avec un dispositif (4), et une seconde configuration dans laquelle le dispositif (4) est retenu par le collier (30), le collier (30) ayant une largeur radiale délimitée entre une surface radialement extérieure et une surface radialement intérieure,
dans lequel l'agencement d'interface de dispositif comprend une oreille qui s'étend radialement vers l'extérieur, l'oreille étant agencée pour se situer dans un évidement (22) disposé dans la surface radialement intérieure du collier (30), le collier (30) comprenant en outre un premier canal (26) qui s'étend suivant la circonférence de la surface radialement intérieure, le premier canal (26) étant en communication avec l'évidement (22) et étant agencé de telle sorte que le collier (30) tourne par rapport à l'agencement d'interface de dispositif entre les première et seconde configurations le premier canal (26) reçoit l'oreille, **caractérisé en ce que** l'évidement comprend un épaulement (46) configuré pour limiter l'introduction du dispositif dans ledit évidement,
dans lequel l'épaulement est agencé pour aligner sensiblement le dispositif avec un second canal (30) disposé dans la surface radialement intérieure du collier (16) et la rotation du collier (16) entre les première et seconde configurations permet au second canal (30) de recevoir le dispositif (4).
